# EUROPEAN PATENT APPLICATION

(11) **EP 4 631 498 A1**
(43) Date of publication of application: **15.10.2025**
(21) Application number: 23907670.6
(22) Date of filing: 19.12.2023
(51) Int. Cl.: A61K 9/107, A61K 47/26, A61K 47/08, A61K 31/351, A61K 9/00, A61P 27/02

(54) **EYE DROP-TYPE PHARMACEUTICAL COMPOSITION CONTAINING ENVOGLIFLOZIN**

(30) Priority: 21.12.2022 KR 20220180554
(71) Applicant: Daewoong Therapeutics Inc., Hwaseong-si, Gyeonggi-do 18469 (KR)
(72) Inventor: KANG, Min Hyung, Yongin-si Gyeonggi-do 17066 (KR); LEE, Eun Seok, Seongnam-si Gyeonggi-do 13640 (KR); PARK, Sang Han, Hwaseong-si Gyeonggi-do 18469 (KR)
(74) Representative: Turner, Craig Robert
(86) International application number: PCT/KR2023/020926
(87) International publication number: WO 2024/136385

(57) **Abstract**

The present invention provides a pharmaceutical composition in the form of an eye drop formulation, comprising enavogliflozin or a pharmaceutically acceptable salt thereof as an active ingredient; and a combination of polysorbate and polyoxyl 40 stearate as a solubilizing agent and a stabilizing agent. The pharmaceutical composition of the present invention comprises a combination of the specific surfactants; and thus can not only remarkably enhance stability but also significantly increase the intraocular exposure of enavogliflozin.

## Description

### TECHNICAL FIELD

The present invention relates to a pharmaceutical composition in the form of an eye drop formulation comprising enavogliflozin. More specifically, the present invention relates to a pharmaceutical composition in the form of an eye drop formulation, comprising enavogliflozin or a pharmaceutically acceptable salt thereof as an active ingredient; and a combination of specific surfactants, which provides excellent stability and increased intraocular exposure.

### BACKGROUND ART

Enavogliflozin, which has a chemical structure of the following Chemical Formula 1, has inhibitory activity against SGLT2 (sodium-dependent glucose cotransporter 2) present in the intestines and kidneys and thus can be usefully used in the treatment of metabolic disorders, especially diabetes (WO 2012/165914, WO 2017/217792, etc.).

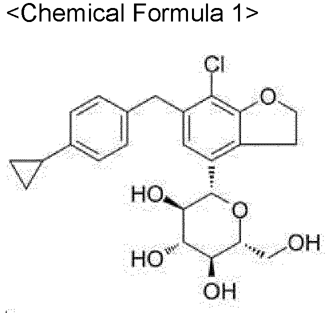

The present inventors have found that a SGLT2 inhibitor including enavogliflozin or a pharmaceutically acceptable salt thereof has excellent preventive or therapeutic activity against diabetic eye diseases (e.g., diabetic retinopathy) (Korean Laid-open Publication No. 10-2022-0079480). And, the present inventors have found that a SGLT2 inhibitor including enavogliflozin or a pharmaceutically acceptable salt thereof has excellent preventive or therapeutic activity against macular degeneration (Korean Laid-open Publication No. 10-2023-0007963).

Enavogliflozin has low water solubility, which makes it difficult to formulate into an aqueous solution form, for example, to an eye drop formulation in the form of an aqueous solution. To solve said problem, Korean Laid-open Publication Nos. 10-2022-0079480 and 10-2023-0007963 disclose examples of the eye drop formulations in which enavogliflozin is solubilized by using polyoxyl 35 castor oil (Kolliphor^{™} ELP) and polysorbate 80 (Tween^{™} 80) as solubilizing agents.

Meanwhile, intraocular drug delivery is required for the treatment of posterior segment eye diseases such as diabetic retinopathy and macular degeneration. Especially, in order for a drug to reach a disease site (e.g., the retina), it is necessary not only to effectively solubilize the drug, but also to increase the intraocular drug exposure by allowing the solubilized drug to penetrate through the cornea or conjunctiva and reach the retina. In addition, since patients with posterior segment eye diseases such as diabetic retinopathy and macular degeneration require long-term repeated administration, it is required to ensure excellent stability without drug precipitation or generation of degradation products from a formulation (e.g., eye drop formulations).

### DISCLOSURE

### Technical Problem

The present inventors have found that the conventional enavogliflozin-containing eye drop formulations (e.g., the eye drop formulations disclosed in Korean Laid-open Publication Nos. 10-2022-0079480 and 10-2023-0007963) exhibit significantly reduced stability upon long-term storage and do not provide satisfactory intraocular exposure. To solve these problems, the present inventors carried out various formulation studies. As a result, the present inventors have found that, when formulation is performed using a combination of specific surfactants, the physical and chemical stabilities of enavogliflozin-containing eye drop formulations are remarkably increased. That is, the present inventors have found that the combination of specific surfactants functions not only as a solubilizing agent but also as a stabilizing agent in an enavogliflozin-containing eye drop formulation. In addition, the present inventors have found that an eye drop formulation obtained by using the combination of specific surfactants can provide excellent pharmacological activity by significantly increasing the intraocular exposure of enavogliflozin.

Therefore, it is an object of the present invention to provide a pharmaceutical composition in the form of an eye drop formulation comprising enavogliflozin or a pharmaceutically acceptable salt thereof and the combination of specific surfactants.

### Technical Solution

According to an aspect of the present invention, there is provided a pharmaceutical composition in the form of an eye drop formulation, comprising enavogliflozin or a pharmaceutically acceptable salt thereof as an active ingredient; and a combination of polysorbate and polyoxyl 40 stearate as a solubilizing agent and a stabilizing agent.

The enavogliflozin or a pharmaceutically acceptable salt thereof may be present at a concentration of 0.1 ~ 10 w/v%, preferably 0.3 ~ 8 w/v%, more preferably 0.5 ~ 5 w/v%.

The combination of polysorbate and polyoxyl 40 stearate may be present at a concentration of 1 ~ 15 w/v%, preferably 4 ~ 10 w/v%. A weight ratio of polysorbate and polyoxyl 40 stearate may be 1 : 1 ~ 10, preferably 1 : 2 ~ 8.

The pharmaceutical composition of the present invention may form nanomicelles having an average particle diameter of 1 ~ 500 nm, preferably 5 ~ 50 nm.

The pharmaceutical composition of the present invention may further comprise at least one excipient selected from the group consisting of a penetration enhancer, a tonicity-adjusting agent, and a pH-adjusting agent. In an embodiment, the penetration enhancer may be D-α-tocopheryl polyethylene glycol succinate. In another embodiment, the tonicity-adjusting agent may be glycerin. In still another embodiment, the pharmaceutical composition of the present invention may have an osmolality of 230 to 350 mOsmol/kg and/or a pH of pH 6.0 ~ pH 7.5.

### ADVANTAGEOUS EFFECTS

The pharmaceutical composition in the form of an eye drop formulation according to the present invention comprises a combination of specific surfactants, i.e., a combination of polysorbate and polyoxyl 40 stearate, which makes it possible to increase the physical and chemical stabilities. Therefore, it has been found by the present invention that the combination of polysorbate and polyoxyl 40 stearate functions not only as a solubilizing agent but also as a stabilizing agent. In addition, the pharmaceutical composition according to the present invention, which comprises said combination of specific surfactants, can provide excellent pharmacological activity by significantly increasing the intraocular exposure of enavogliflozin.

### DESCRIPTION OF DRAWINGS

FIG. 1 shows the results obtained by measuring the saturation solubility of enavogliflozin according to the surfactant mixtures (the mixtures of Tween^{™} 80 and Kolliphor^{™} EL).
FIG. 2 shows the appearances at the Dissolution-1 step (40°C), at the Dissolution-2 step (80°C), and at the Filtration step (25°C), during the preparation of Formulation-1.
FIG. 3a, FIG. 3b, and FIG. 3c show the results obtained by analyzing the particle size distributions of Formulation-3 (FIG. 3a), Formulation-4 (FIG. 3b), and Formulation-5 (FIG. 3c), respectively.
FIG. 4 shows the drug concentration profiles in the rat eyes obtained by performing the pharmacokinetic study.

### BEST MODE FOR CARRYING OUT THE INVENTION

The present invention provides a pharmaceutical composition in the form of an eye drop formulation, comprising enavogliflozin or a pharmaceutically acceptable salt thereof as an active ingredient; and a combination of polysorbate and polyoxyl 40 stearate as a solubilizing agent and a stabilizing agent.

The pharmaceutical composition of the present invention may comprise the enavogliflozin or a pharmaceutically acceptable salt thereof in an amount suitable for the treatment of posterior segment eye diseases such as diabetic retinopathy and macular degeneration. For example, the enavogliflozin or a pharmaceutically acceptable salt thereof may be present at a concentration of 0.1 ~ 10 w/v%, preferably 0.3 ~ 8 w/v%, more preferably 0.5 ~ 5 w/v%, in the pharmaceutical composition.

It has been found by the present invention that, when formulation is performed using a combination of specific surfactants, i.e., a combination of polysorbate and polyoxyl 40 stearate, the physical and chemical stabilities of enavogliflozin-containing eye drop formulations can be remarkably increased. In addition, it has been found by the present invention that the pharmaceutical composition of the present invention, which comprises said combination of specific surfactants, can provide excellent pharmacological activity by significantly increasing the intraocular exposure of enavogliflozin.

The polysorbate may be polysorbate 20, 40, 80, etc., and preferably polysorbate 80. The polyoxyl 40 stearate is also referred to as polyethylene glycol monostearate. In the pharmaceutical composition of the present invention, the combination of polysorbate and polyoxyl 40 stearate may be present at a concentration of 1 ~ 15 w/v%, preferably 4 ~ 10 w/v%, in the pharmaceutical composition. A weight ratio of polysorbate and polyoxyl 40 stearate may be 1 : 1 ~ 10, preferably 1 : 2 ~ 8. In an embodiment, the polysorbate may be present at a concentration of 0.1 ~ 10 w/v%, preferably 1 ~ 4 w/v%, in the pharmaceutical composition; the polyoxyl 40 stearate may be present at a concentration of 0.1 ~ 10 w/v%, preferably 3 ~ 7 w/v%, in the pharmaceutical composition, within the weight ratio in the above. The pharmaceutical composition of the present invention comprises the combination of polysorbate and polyoxyl 40 stearate and thus forms nanomicelles having an average particle diameter of 1 ~ 500 nm, preferably 5 ~ 50 nm.

The pharmaceutical composition of the present invention may comprise excipients conventionally used in the field of eye drop formulations. For example, the pharmaceutical composition of the present invention may further comprise at least one excipient selected from the group consisting of a penetration enhancer, a tonicity-adjusting agent, and a pH-adjusting agent. In an embodiment, the penetration enhancer may be D-α-tocopheryl polyethylene glycol succinate (TPGS), which may be present e.g., at a concentration of 0.1 ~ 0.5 w/v%. In another embodiment, the tonicity-adjusting agent may be glycerin, which may be present e.g., at a concentration of 0.5 ~ 6 w/v%. In still another embodiment, the pharmaceutical composition of the present invention may have an osmolality of 230 to 350 mOsmol/kg and/or a pH of pH 6.0 ~ pH 7.5.

In an embodiment of the present invention, there is provided a pharmaceutical composition, comprising 0.5 ~ 5 w/v% of enavogliflozin or a pharmaceutically acceptable salt thereof; 4 ~ 10 w/v% of the combination of polysorbate and polyoxyl 40 stearate; 0.1 ~ 0.5 w/v% of D-α-tocopheryl polyethylene glycol succinate; 0.5 ~ 6 w/v% of glycerin; and a pH-adjusting agent, in an aqueous medium, and forming nanomicelles having an average particle diameter of 5 ~ 50 nm. In said embodiment, a weight ratio of polysorbate and polyoxyl 40 stearate may be 1 : 2 ~ 8.

Hereinafter, the present invention will be described in more detail through Examples. However, these Examples are provided for illustration purposes only, and are not intended to limit the scope of the invention.

### Example 1: Formulation Study - 1

### (1) Solubility Evaluation

Using the FDA inactive ingredient database (https://www.accessdata.fda.gov/scripts/cder/iig/index.cfm), the excipients available in the route of ophthalmic administration were dissolved in purified water at the respective maximum concentration recommended by the FDA, and then enavogliflozin was dissolved until saturation was achieved therein, thereby measuring the saturation solubilities.

### (1-1) Preparation of standard solutions

According to Table 1 below, enavogliflozin in the amount corresponding to each standard solution and 70 mL of methanol were placed in a volumetric flask and then completely dissolved under ultrasonic agitation. Methanol was added thereto to adjust the total volume to 100 mL. The resulting solution was taken and filtered through a 0.45 µm RC (Regenerated Cellulose) membrane filter. The first 2 mL was discarded and then the filtrate was used as a standard solution.

**Table 1**

| | Concentration (µg/mL) | Taken amount of enavogliflozin (mg) |
|---|---|---|
| Standard solution 1 | 20.0 | 2.0 |
| Standard solution 2 | 70.0 | 7.0 |
| Standard solution 3 | 100.0 | 10.0 |
| Standard solution 4 | 150.0 | 15.0 |
| Standard solution 5 | 250.0 | 25.0 |

### (1-2) Preparation of test solutions

Each excipient was dissolved in 5 mL of purified water at the maximum concentration recommended by the FDA (Table 2) and then enavogliflozin was added thereto until it was no longer dissolved, followed by stirring for approximately 12 hours. After confirming no dissolution after 12 hours, each sample was centrifuged at 4000 rpm for 30 minutes and the clear supernatant (1 mL) was taken and then diluted with methanol. The resulting solution was filtered through a 0.45 µm RC membrane filter. The first 2 mL was discarded and then the filtrate was used as a test solution.

### (1-3) HPLC Analysis

The peak areas of the standard and test solutions were analyzed under the following HPLC conditions.
- Detector: UV spectrophotometer (wavelength: 225 nm)
- Column: Capcellpak C18 (4.6 x 250 mm, 5 µm)
- Column temperature: about 35°C
- Sample temperature: about 25°C
- Mobile phase: Buffer solution : acetonitrile = 25 : 75 (v/v)
   (Buffer solution: prepared by dissolving 100 µl of trifluoroacetic acid in 1000 mL of water)
- Flow rate: 1.0 mL/min
- Injection volume: 10 µL
- Analysis time: about 20 minutes

### (1-4) Solubility for each excipient

The results obtained by measuring the saturation solubility of enavogliflozin for each excipient are shown in Table 2 below.

**Table 2**

| | Excipient | Concentration of excipient (%w/v) | Saturation solubility (mg/mL) | Solubility for unit concentration (mg/%) |
|---|---|---|---|---|
| 1 | Aqualon^{™} CMC | 0.50 | 0.30 | 0.59 |
| 2 | Glycerol | 2.25 | 0.24 | 0.11 |
| 3 | Cavasol^{™} W8 | 1.50 | 1.09 | 0.72 |
| 4 | HPMC 606 | 0.60 | 0.33 | 0.56 |
| 5 | PEG 400 | 4.00 | 0.33 | 0.08 |
| 6 | Kolliphor^{™} HS 15 | 0.25 | 1.02 | 4.09 |
| 7 | Kolliphor^{™} RH 40 | 1.00 | 3.64 | 3.64 |
| 8 | Kollidon^{™} K30 | 15.00 | 3.60 | 0.24 |
| 9 | Kollidon^{™} K90 | 1.50 | 0.62 | 0.41 |
| 10 | Kollisolv^{™} PG | 1.50 | 0.27 | 0.18 |
| 11 | EtOH | 0.50 | 0.26 | 0.51 |
| 12 | Tween^{™} 20 | 0.05 | 0.36 | 7.21 |
| 13 | TPGS | 0.50 | 2.31 | 4.63 |
| 14 | Kolliphor^{™} EL | 5.00 | 16.84 | 3.37 |
| 15 | Tween^{™} 80 | 4.00 | 15.80 | 3.95 |

From the results of Table 2 above, it can be seen that the improvement in solubility of enavogliflozin was the highest for Tween^{™} 80 and Kolliphor^{™} EL. The improvement in solubility of enavogliflozin for TPGS, a penetration enhancer, was also relatively high. Although the surfactants, i.e., Kolliphor^{™} HS 15, Tween^{™} 20, and Kolliphor^{™} RH 40 also showed similar solubilizing effects, these showed drawbacks in that the maximum amounts capable of using as an ophthalmic excipient were limited.

### (2) Evaluation of saturation solubility according to the concentrations of surfactant

According to the results of (1) in the above, the surfactants Tween^{™} 80 and Kolliphor^{™} EL were selected as excipients for solubilizing enavogliflozin. The target concentration of enavogliflozin in an eye drop formulation is 0.5 to 5.0 w/v%, but the saturation solubility does not reach said concentration. Therefore, the two components having the best solubility were mixed and the saturation solubility of the active ingredient was measured according to the ratios thereof.

### (2-1) Test method

Tween^{™} 80 and Kolliphor^{™} EL were mixed in the ratio shown in Table 3 below. The resulting mixture was heated to 40°C and then enavogliflozin was dissolved by adding thereto in a certain amount. The point at which enavogliflozin was no longer dissolved and the appearance thereof becomes cloudy was defined as a saturation solubility.

**Table 3**

| | Surf mix-1 | Surf mix-2 | Surf mix-3 | Surf mix-4 |
|---|---|---|---|---|
| Tween^{™} 80 | 0.33% | 1.0% | 1.5% | 2.0% |
| Kolliphor^{™} EL | 0.66% | 2.0% | 3.0% | 4.0% |
| Total concentration of surfactants (%) | 1.0 | 3.0 | 4.5 | 6.0 |

### (2-2) Test results

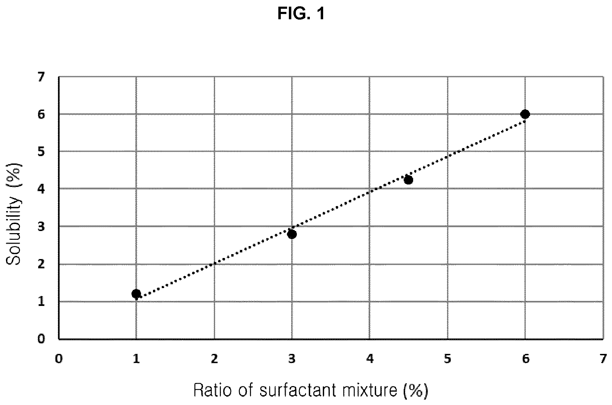

The saturation solubility was analyzed by adding the amounts of enavogliflozin dissolved up to the point where the appearance was maintained. As can be seen from the results of FIG. 1, the solubility of enavogliflozin increased proportionally as the total concentration of surfactants increased and the surfactants need to be used at a concentration of at least 5.0 w/v% for dissolving to the target concentration of 5.0 w/v%.

### (3) Preparation and evaluation of formulations

### (3-1) Preparation of formulations

Based on the test results above, enavogliflozin-containing eye drop formulations (Formulation-1: 5.0 w/v% and Formulation-2: 2.0 w/v%) were prepared according to the components and amounts of Table 4 below. Tween^{™} 80 and Kolliphor^{™} EL were used as surfactants and TPGS was used as a penetration enhancer, and glycerin was used as a tonicity-adjusting agent. Specifically, the excipients (Tween^{™} 80, Kolliphor^{™} EL, glycerin, anhydrous sodium dihydrogen phosphate, and sodium hydroxide) and water for injection were heated to about 40°C under stirring (Dissolution-1 step). The active ingredient (enavogliflozin) was added thereto and heated to approximately 80°C (Dissolution-2 step). The solution was then cooled to 25°C and filtered through a 0.2 µm RC, PTFE, or PVDF membrane filter (Filtration step). Each obtained eye drop formulation was filled into a polyethylene tube.

**Table 4**

| | Raw material | Formulation-1 (Active ingredient: 5.0 w/v%) | Formulation-2 (Active ingredient: 2.0 w/v%) |
|---|---|---|---|
| | | Amount (mg/mL) | Amount (mg/mL) |
| Active ingredient | Enavogliflozin | 50.0 | 20.0 |
| Surfactant | Tween^{™} 80 | 20.0 | 15.0 |
| Surfactant | Kolliphor^{™} EL | 40.0 | 30.0 |
| Penetration enhancer | TPGS | 5.0 | 5.0 |
| Tonicity-adjusting agent | Glycerin | 25.0 | 20.0 |
| pH-adjusting agent | Anhydrous sodium dihydrogen phosphate | 0.12 | 0.12 |
| pH-adjusting agent | Sodium hydroxide | q.s. | q.s. |
| Solvent | Water for injection | q.s. | q.s. |

### (3-2) Evaluation of formulations

The membrane filter made of RC, PTFE, or PVDF as a filter material was used for filtration and the contents of the active ingredient before and after filtration were analyzed by HPLC in the same manner as in (1) above to confirm whether the active ingredient was adsorbed on the filter. The results are shown in Table 5 below.

**Table 5**

| | Filter material | Before filtration (%) | After filtration (%) | Adsorption rate (%) |
|---|---|---|---|---|
| Formulation-1 | RC | 98.9 | 98.8 | 0.1 |
| | PTFE | | 98.5 | 0.4 |
| | PVDF | | 98.9 | 0.0 |
| Formulation-2 | RC | 101.7 | 102.0 | -0.3 |
| | PTFE | | 101.9 | -0.2 |
| | PVDF | | 101.3 | 0.4 |

As can be seen from the results of Table 5 above, the active ingredient did not show significant adsorption regardless of the filter material of the filter used in the filtration step and the most conventionally used RC material was selected as a filter for filtration step.

In addition, the appearance, content, pH, and osmolality of Formulation-1 and Formulation-2 were evaluated. The appearance was confirmed with the naked eye and the content was analyzed by HPLC analysis using the same method as (1) above. The pH was measured using the Metrohm 913 instrument and the osmolality was measured using the OSMOMAT 3000D instrument. The results obtained by evaluating the formulation as described above are as shown in Table 6 below.

**Table 6**

| | Target criteria | Formulation-1 | Formulation-2 |
|---|---|---|---|
| Appearance | - | Clear Liquid with soft bluish light | Colorless clear liquid |
| pH | 6.5~8.0 | 6.6 | 6.6 |
| Osmolality (mOsmol/kg) | 230~350 | 354 (unsuitable) | 276 |
| Content (%) | 95.0~105.0 | 99.0 | 100.8 |

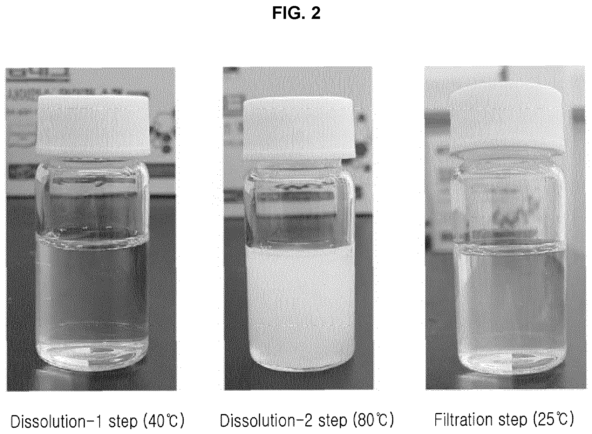

During the process of dissolving the active ingredient, the phenomenon was briefly observed where the active ingredient was not sufficiently dissolved and became suspended when heated. This would originate from a phenomenon in which the active ingredient is precipitated at a temperature above the cloud point of the nonionic surfactants. It is considered that, when cooled to a temperature below the cloud point in the subsequent cooling step, the surfactants were reassociated to form nanomicelles, thereby dissolving the active ingredient (see FIG. 2).

The osmolality of Formulation-1 slightly exceeded the target criteria and thus it would be required to control the amount of tonicity-adjusting agent. Although the osmolality of Formulation-1 did not meet the target criteria, it was used as a sample in Formulation Study - 2 of Example 2 to observe the tendency of degradation products.

### Example 2: Formulation Study - 2

### (1) Preparation of formulations

Based on the test results of Example 1, the eye drop formulations were prepared using the same method as (3-1) of Example 1 (filtration using an RC membrane filter) according to the components and amounts of Table 7, with the active ingredient in the concentrations of 1.0 w/v%, 3.0 w/v%, and 5.0 w/v%, respectively. Considering that the eye drop formulation with 1.0 w/v% of the active ingredient would be sufficient in terms of saturation solubility, it was prepared by reducing the amount of surfactants by 30%. In addition, the amounts of the pH-adjusting agent and the tonicity-adjusting agent was adjusted so as to be suitable for the pH or the osmolality criteria (Table 7).

**Table 7**

| | Raw material | Formulation-3 (Active ingredient: 5.0 w/v%) | Formulation-4 (Active ingredient: 3.0 w/v%) | Formulation-5 (Active ingredient: 1.0 w/v%) |
|---|---|---|---|---|
| | | Amount (mg/mL) | Amount (mg/mL) | Amount (mg/mL) |
| Active ingredient | Enavogliflozin | 50.0 | 30.0 | 10.0 |
| Surfactant | Tween^{™} 80 | 20.0 | 20.0 | 13.33 |
| Surfactant | Kolliphor^{™} EL | 40.0 | 40.0 | 26.66 |
| Penetration enhancer | TPGS | 5.0 | 5.0 | 5.0 |
| Tonicity-adjusting | Glycerin | 20.0 | 20.0 | 20.0 |
| agent | | | | |
| pH-adjusting agent | Anhydrous sodium dihydrogen phosphate | 0.12 | 0.12 | 0.12 |
| pH-adjusting agent | Sodium hydroxide | q.s. | q.s. | q.s. |
| Solvent | Water for injection | q.s. | q.s. | q.s. |

### (2) Evaluation of appearance, content, pH, and osmolality

The appearance, content, pH, and osmolality of Formulation-3, Formulation-4, and Formulation-5 were evaluated using the same method as (3-2) of Example 1. The results are shown in Table 8 below.

**Table 8**

| | Target criteria | Formulation-3 | Formulation-4 | Formulation-5 | Suitability |
|---|---|---|---|---|---|
| Appearance | - | Clear Liquid with soft bluish light | Colorless clear liquid | Colorless clear liquid | - |
| pH | 6.5~8.0 | 6.6 | 6.6 | 6.6 | Suitable |
| Osmolality (mOsmol/kg) | 230~350 | 341 | 317 | 312 | Suitable |
| Content (%) | 95.0~105.0 | 99.6 | 99.1 | 99.3 | Suitable |

From the results of Table 8 above, the formulations of Formulation-3, Formulation-4, and Formulation-5 were found to meet the target criteria. The pHs were measured to be almost the same at all concentrations and thus the pH-adjusting agent would be sufficiently performing its buffering function. Additionally, the osmolality did not increase in proportion to the amount of active ingredient or surfactants.

### (3) Evaluation of particle size distribution and zeta potential

Tween^{™} 80 and Kolliphor^{™} EL, which are the solubilizing agents used for achieving the target concentration of active ingredient (i.e., enavogliflozin,) form micelles at a concentration above the critical micelle concentration. Since the formation of micelles or nanomicelles may affect the efficiency of drug delivery, we investigate whether the formulations of Formulation-3, Formulation-4, and Formulation-5 were form nanomicelles. The particle size distribution formed in the solution was analyzed using dynamic light scattering (DLS). The zeta potential was also measured to confirm the charge formed on the particle surface. The ELSZ -2000 of Otsuka was used as a DLS measurement instrument and the measurement was carried out under the conditions shown in Table 9 below.

**Table 9**

| | Particle size distribution | Zeta potential |
|---|---|---|
| Cell type | Disposable cell | Flow cell |
| Diluent | Water | Water |
| Temperature (°C) | 25.0 | 25.0 |
| Analytical method | Marquardt | Smoluchowski |

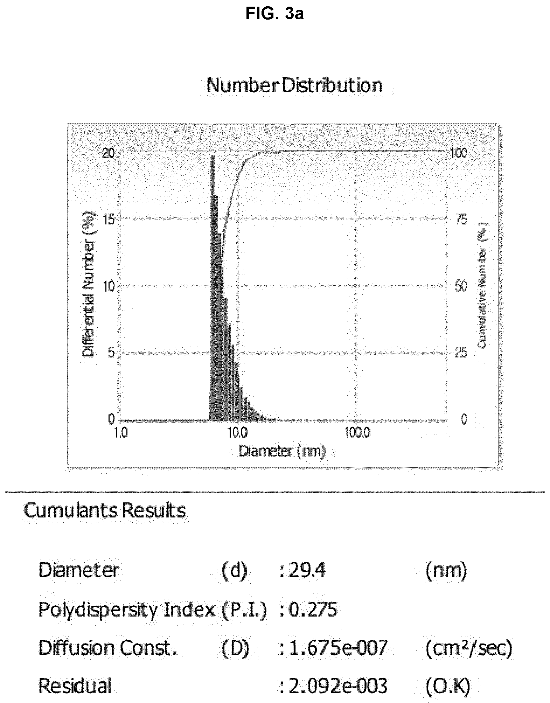

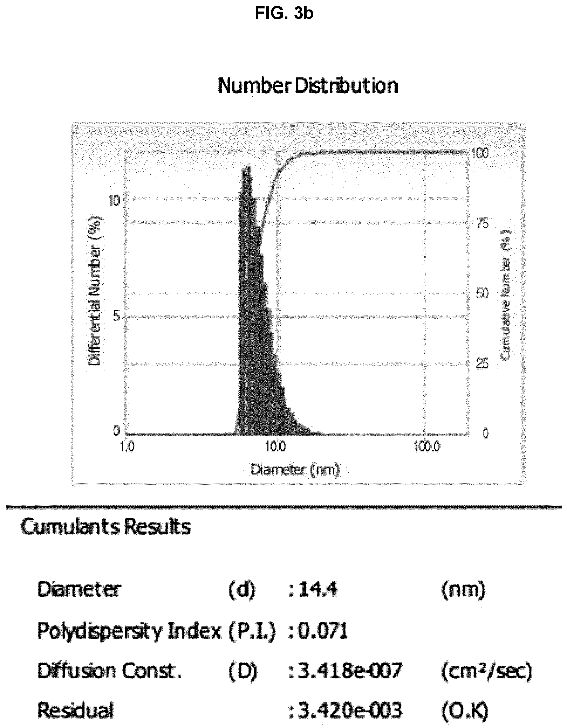

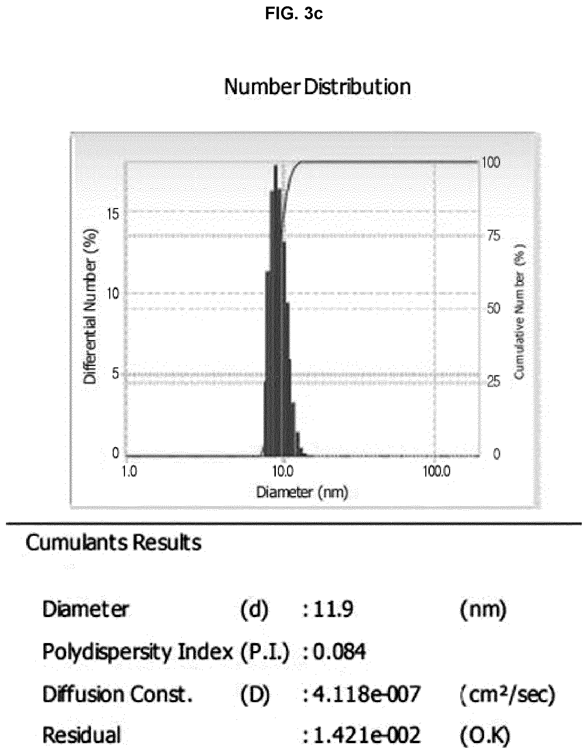

The results obtained by analyzing the particle size distributions of the formulations of Formulation-3, Formulation-4, and Formulation-5 are as shown in Table 10 and FIG. 3 (i.e., FIG. 3a, FIG. 3b, and FIG. 3c). And, the results obtained by measuring the zeta potentials thereof are as shown in Table 10 below.

**Table 10**

| | Formulation-3 (5.0 w/v%) | Formulation-4 (3.0 w/v%) | Formulation-5 (1.0 w/v%) |
|---|---|---|---|
| Size (nm, Z-AVR) | 29.4 | 14.4 | 11.9 |
| Size (nm, No. of distribution) | 7.9+±2.2 | 7.8±2.0 | 9.9+±1.2 |
| Zeta potential | -8.88 | -3.20 | -23.80 |

As can be seen from the results of Table 10 above, the average particle size (Z-average) ranged from 11.9 ~ 29.4 nm and showed a tendency to increase as the ratio of the active ingredient increased. However, based on the number of distributed particles (No. of distribution), all formulations showed particle sizes of less than 10 nm. The zeta potential was found to have a weak negative charge.

### Example 3: Formulation Study - 3

### (1) Stability Evaluation

The results obtained by evaluating the stability of Formulation-2 containing the combination of Kolliphor^{™} EL and Tween^{™} 80, while storing under an accelerated condition and under a room temperature condition for 2 months and for 4 months, are as shown in Table 11 below.

**Table 11**

| | Initial | 2 Months at room temperature | 4 Months at room temperature | 2 Months under accelerated condition | 4 Months under accelerated condition |
|---|---|---|---|---|---|
| Appearance | Colorless clear liquid | Colorless clear liquid | Colorless clear liquid | Colorless clear liquid | Colorless clear liquid |
| Content | 99.0% | 96.8% | 86.6% | 64.8% | 60.2% |
| Total degradation product | 0.11% | 0.26% | 0.24% | 0.12% | 0.58% |
| Suitability | Suitable | Suitable | Unsuitable | Unsuitable | Unsuitable |

As can be seen from the results of Table 11 above, Formulation-2 was unsuitable as it exhibited precipitation or a content below the standard criteria in the stability tests conducted at the room temperature condition for 4 months, under the accelerated condition for 2 months, and under the accelerated condition for 4 months. When a decrease in content occurs due to degradation of the active ingredient, the amount of degradation products increases significantly. However, the increase in degradation products derived from enavogliflozin was not significant.

### (2) Improvement of formulation

Since the solubility of poorly soluble enavogliflozin was improved by using the surfactants, there would be a possibility that the interaction between the active ingredient and the surfactants may affect the stability during the storage thereof. Therefore, it can be indirectly evaluated by measuring the cloud point that solubility rapidly decreases when the water-soluble group in a nonionic surfactant is dehydrated upon heating. The reason for instability of the formulations was thus investigated and further formulation study involving the modification of solubilizing agents was carried out in order to stabilize the formulations.

### (2-1) Evaluation on solubilization by polyoxyl 40 stearate

Polyoxyl 40 stearate (Myrj^{™} S40) was dissolved in 5 mL of purified water at the concentration of 7.0 w/v% and then enavogliflozin was added thereto until it was no longer dissolved, followed by stirring for approximately 12 hours. Thereafter, the degree of solubilization was evaluated in the same manner as in Example 1.

### (2-2) Measurement of cloud point

The cloud point was measured by observing in naked eyes the point at which the appearance became opaque while gradually heating the test formulations. 5 mL of the test formulations (i.e., the formulations of Formulation-6, Formulation-7, and Formulation-8 prepared in (2-3) below and the formulation of Formulation-1) was placed in a transparent glass vial and then stirred in a water bath while increasing the temperature by 0.5°C each time. The temperature at which the appearance of the formulation was changed from a clear and transparent liquid to a turbid suspension was taken as a cloud point.

### (2-3) Preparation of formulations

The eye drop formulations were prepared using the same method as (3-1) of Example 1 according to the components and amounts of Table 12, with the active ingredient in the concentrations of 1.0 w/v%, 3.0 w/v%, and 5.0 w/v%, respectively.

**Table 12**

| | Raw material | Formulation-6 (Active ingredient: 1.0 w/v%) | Formulation-7 (Active ingredient: 3.0 w/v%) | Formulation-8 (Active ingredient: 5.0 w/v%) |
|---|---|---|---|---|
| | | Amount (mg/mL) | Amount (mg/mL) | Amount (mg/mL) |
| Active ingredient | Enavogliflozin | 10.0 | 30.0 | 50.0 |
| Surfactant | Tween^{™} 80 | 20.0 | 20.0 | 20.0 |
| Surfactant | Polyoxyl 40 stearate | 70.0 | 70.0 | 70.0 |
| Penetration enhancer | TPGS | 5.0 | 5.0 | 5.0 |
| Tonicity-adjusting agent | Glycerin | 16.0 | 16.0 | 16.0 |
| pH-adjusting agent | Anhydrous sodium dihydrogen phosphate | 0.156 | 0.156 | 0.156 |
| pH-adjusting agent | Sodium hydroxide | q.s. | q.s. | q.s. |
| Solvent | Water for injection | q.s. | q.s. | q.s. |

### (2-4-1) Evaluation on solubilization by polyoxyl 40 stearate

According to the FDA-recommended examples for using an excipient, Kolliphor^{™} EL can be used at a concentration of up to 5.0 w/v% and polyoxyl 40 stearate can be used at a concentration of up to 7.0 w/v%, for the route of ophthalmic administration. Based on these, the results obtained by comparing the solubilizing levels of the two excipients are as shown in Table 13 below.

**Table 13**

| Excipient | Concentration of excipient | Saturation solubility of enavogliflozin (mg/mL) |
|---|---|---|
| Polyoxyl 40 stearate | 7.0 w/v% | 32.01 |
| Kolliphor^{™} EL | 5.0 w/v% | 16.84 |

As can be seen from the results of Table 13 above, considering the maximum amount, polyoxyl 40 stearate can solubilize enavogliflozin about twice as much as Kolliphor^{™} EL.

### (2-4-2) Evaluation of appearance, content, pH, osmolality, and cloud point

Based on the above results, Kolliphor^{™} EL was changed to polyoxyl 40 stearate to prepare the formulations of Formulation-6, Formulation-7, and Formulation-8. The appearance, content, pH, osmolality, and cloud point thereof were evaluated. For comparison, the cloud point of the formulation of Formulation-1 was measured. The results are shown in Table 14 below.

**Table 14**

| | Formulation-1 (5.0 wt/v/%) | Formulation-6 (1.0 wt/v/%) | Formulation-7 (3.0 wt/v/%) | Formulation-8 (5.0 wt/v/%) |
|---|---|---|---|---|
| Appearance | Clear Liquid with soft bluish light | Colorless clear liquid | Colorless clear liquid | Colorless clear liquid |
| pH | 6.6 | 6.7 | 6.7 | 6.8 |
| Osmolality (mOsmol/kg) | 354 | 292 | 348 | 318 |
| Content (%) | 99.0 | 103.1 | 103.1 | 101.7 |
| Cloud point (°C) | 35 | Not observed up to 90°C | Not observed up to 90°C | 74 |
| Note | Kolliphor^{™} EL used | Polyoxyl 40 stearate used | | |

As can be seen from the results of Table 14, when comparing Formulation-1 and Formulation-8 that have the same active ingredient concentration, the cloud point was found to increase by approximately 40°C. This result shows that polyoxyl 40 stearate maintains its solubilizing ability even when dehydration of the water-soluble portion occurs to some extent according to heating, in contrast to Kolliphor^{™} EL. The formulations of Formulation-6, Formulation-7, and Formulation-8 showed suitable values in all evaluation items.

### (2-4-3) Stability Evaluation

The results obtained by evaluating the stability of the formulations, i.e., Formulation-1, Formulation-2, Formulation-6, Formulation-7, and Formulation-8 by measuring the contents of the active ingredient, while storing under an accelerated condition for 6 months, are as shown in Table 15 below.

**Table 15**

| | Concentration of the active ingredient | Initial (%) | 2 Months under accelerated condition (%) | 4 Months under accelerated condition (%) | 6 Months under accelerated condition (%) |
|---|---|---|---|---|---|
| Formulation-1 | 5.0 w/v% | 99.00 | 64.78 | 60.16 | 7.35 |
| Formulation-2 | 2.0 w/v% | 100.80 | 64.80 | 60.20 | 13.52 |
| Formulation-6 | 1.0 w/v% | 103.14 | 100.06 | - | 97.35 |
| Formulation-7 | 3.0 w/v% | 103.09 | 98.85 | - | 98.03 |
| Formulation-8 | 5.0 w/v% | 101.66 | 98.50 | - | 99.61 |

As can be seen from the results of Table 15, while the formulation of Formulation Example-1 showed a content decrease of more than 90% when stored for 6 months under the accelerated condition, the formulation of Formulation-8 containing the same concentration showed remarkably excellent stability. The low-concentration formulations, i.e., the formulations of Formulation-6 and Formulation-7, also exhibited remarkably excellent stability. Therefore, it can be confirmed that the use of polyoxyl 40 stearate can remarkably improve the stability of enavogliflozin-containing eye drop formulations.

### Example 4: Formulation Study-4

### (1) Preparation of formulations

Based on the test results above, the eye drop formulations were prepared using the same method as (3-1) of Example 1 according to the components and amounts of Table 16, with the active ingredient in the concentration of 2.0 w/v%.

**Table 16**

| | Raw material | Formulation-9 (Active ingredient: 2.0 w/v%) | Formulation-10 (Active ingredient: 2.0 w/v%) |
|---|---|---|---|
| | | Amount (mg/mL) | Amount (mg/mL) |
| Active ingredient | Enavogliflozin | 20.0 | 20.0 |
| Surfactant | Tween^{™} 80 | 20.0 | 20.0 |
| Surfactant | Kolliphor^{™} EL | 40.0 | - |
| Surfactant | Polyoxyl 40 stearate | - | 70.0 |
| Penetration enhancer | TPGS | 5.0 | 5.0 |
| Tonicity-adjusting agent | Glycerin | 25.0 | 16.0 |
| pH-adjusting agent | Anhydrous sodium dihydrogen phosphate | 0.12 | 0.156 |
| pH-adjusting agent | Sodium hydroxide | q.s. | q.s. |
| Solvent | Water for injection | q.s. | q.s. |

### (2) Stability Evaluation

The results obtained by evaluating the stability of the formulations of Formulation-9 and Formulation-10 by measuring the content of the active ingredient and the content of the total degradation products, while storing under an accelerated condition for 6 months, are as shown in Table 17 below.

**Table 17**

| | Evaluation items | Initial (%) | 6 Months under accelerated condition (%) |
|---|---|---|---|
| Formulation-9 | Content (%) | 100.80 | 92.35 |
| | Total degradation products (%) | 0.09 | 4.98 |
| Formulation-10 | Content (%) | 100.00 | 100.8 |
| | Total degradation | 0.10 | 1.05 |
| | products (%) | | |

From the results of Table 17 above, it can be confirmed that the use of polyoxyl 40 stearate according to the present invention exhibited more excellent stability.

### (3) Pharmacokinetic studies in Rat Eye

To compare the intraocular exposure between the formulations in rats, ocular instillation was carried out. Each formulation was administered to 10 animals. After the rats were stabilized outside the cage, the upper eyelid and the lower eyelid were opened and 5 µL of each test formulation was instilled into each of the right and left eyes using a pipette. The rats were maintained for a certain period of time so as not to flow out after the instillations. At the sampling time points corresponding to 1, 2, 4, 6, and 12 hours after the instillations, each two rats were inhaled-anesthetized with isoflurane and the left and right eyes were extracted using surgical scissors and forceps. The retinal tissues isolated from the left and right eyes were collected into a 1.5 mL Eppendorf tube immediately after the isolation. The pooled retinal tissues placed in a 1.5 mL Eppendorf tube, which were recovered from each subject at each sampling point, were weighed and then immediately frozen in liquid nitrogen. The concentrations of enavogliflozin in the retina were analyzed using LC-MS/MS.

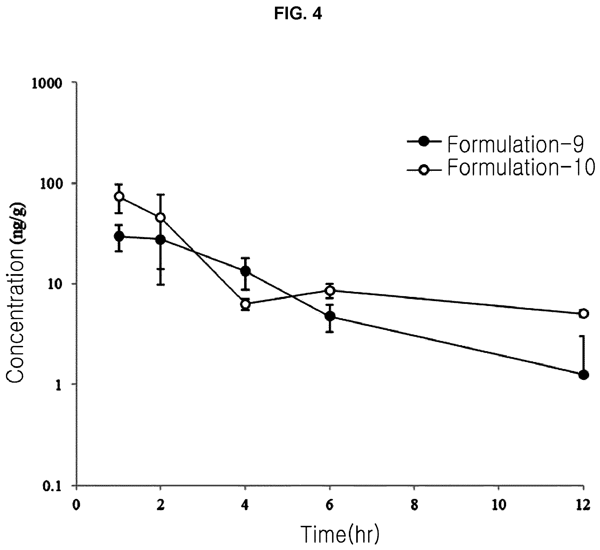

The intraocular drug concentration profiles obtained by performing the pharmacodynamic study in rat eyes as described above are as shown in FIG. 4. As can be seen from the results of FIG. 4, the eye drop formulation obtained by using polyoxyl 40 stearate according to the present invention maintained a significantly higher intraocular concentration. Therefore, the eye drop formulation obtained according to the present invention can increase the intraocular exposure of enavogliflozin, thereby providing excellent pharmacological activity.

## Claims

1. A pharmaceutical composition in the form of an eye drop formulation, comprising enavogliflozin or a pharmaceutically acceptable salt thereof as an active ingredient; and a combination of polysorbate and polyoxyl 40 stearate as a solubilizing agent and a stabilizing agent.

2. The pharmaceutical composition as claimed in claim 1, wherein the enavogliflozin or a pharmaceutically acceptable salt thereof is present at a concentration of 0.1 ~ 10 w/v%.

3. The pharmaceutical composition as claimed in claim 1, wherein the enavogliflozin or a pharmaceutically acceptable salt thereof is present at a concentration of 0.3 ~ 8 w/v%.

4. The pharmaceutical composition as claimed in claim 1, wherein the enavogliflozin or a pharmaceutically acceptable salt thereof is present at a concentration of 0.5 ~ 5 w/v%.

5. The pharmaceutical composition as claimed in claim 1, wherein the combination of polysorbate and polyoxyl 40 stearate is present at a concentration of 1 ~ 15 w/v%.

6. The pharmaceutical composition as claimed in claim 1, wherein the combination of polysorbate and polyoxyl 40 stearate is present at a concentration of 4 ~ 10 w/v%.

7. The pharmaceutical composition as claimed in claim 1, wherein a weight ratio of polysorbate and polyoxyl 40 stearate is 1 : 1 ~ 10.

8. The pharmaceutical composition as claimed in claim 1, wherein a weight ratio of polysorbate and polyoxyl 40 stearate is 1 : 2 ~ 8.

9. The pharmaceutical composition as claimed in claim 1, forming nanomicelles having an average particle diameter of 1 ~ 500 nm.

10. The pharmaceutical composition as claimed in claim 1, forming nanomicelles having an average particle diameter of 5 ~ 50 nm.

11. The pharmaceutical composition as claimed in any one of claims 1 to 10, further comprising at least one excipient selected from the group consisting of a penetration enhancer, a tonicity-adjusting agent, and a pH-adjusting agent.

12. The pharmaceutical composition as claimed in claim 11, wherein the penetration enhancer is D-α-tocopheryl polyethylene glycol succinate.

13. The pharmaceutical composition as claimed in claim 11, wherein the tonicity-adjusting agent is glycerin.

14. The pharmaceutical composition as claimed in claim 13, having an osmolality of 230 to 350 mOsmol/kg.

15. The pharmaceutical composition as claimed in claim 11, having a pH of pH 6.0 ~ pH 7.5.

16. The pharmaceutical composition as claimed in claim 1,
comprising 0.5 ~ 5 w/v% of enavogliflozin or a pharmaceutically acceptable salt thereof; 4 ~ 10 w/v% of the combination of polysorbate and polyoxyl 40 stearate; 0.1 ~ 0.5 w/v% of D-α-tocopheryl polyethylene glycol succinate; 0.5 ~ 6 w/v% of glycerin; and a pH-adjusting agent, in an aqueous medium, and
forming nanomicelles having an average particle diameter of 5 ~ 50 nm.

17. The pharmaceutical composition as claimed in claim 16, wherein the weight ratio of polysorbate and polyoxyl 40 stearate is 1 : 2 ~ 8.
